Europäisches Patentamt

European Patent Office

Office européen des brevets

(19)

(11) **EP 1 502 577 A2**

(12) **EUROPÄISCHE PATENTANMELDUNG**

(43) Veröffentlichungstag:
**02.02.2005 Patentblatt 2005/05**

(51) Int Cl.[7]: **A61K 7/06**

(21) Anmeldenummer: **04405397.3**

(22) Anmeldetag: **25.06.2004**

(84) Benannte Vertragsstaaten:
**AT BE BG CH CY CZ DE DK EE ES FI FR GB GR
HU IE IT LI LU MC NL PL PT RO SE SI SK TR**
Benannte Erstreckungsstaaten:
**AL HR LT LV MK**

(30) Priorität: **30.07.2003 CH 13262003**

(71) Anmelder: **Mibelle AG
5033 Buchs (CH)**

(72) Erfinder:
• **Karlen, Thomas
5000 Aarau (CH)**
• **Hanay, Christiane
5000 Aarau (CH)**

(74) Vertreter: **Rottmann, Maximilian R.
Rottmann, Zimmermann + Partner AG
Glattalstrasse 37
8052 Zürich (CH)**

(54) **Haarfestigendes Mittel**

(57)     Haarfestigende Mittel, welche einen Gehalt an (A) mindestens einem Isobutylen/ Ethylmaleimid/Hydroxyethylmaleimid-Terpolymer und (B) mindestens einem mit (A) verträglichen kationischen Polymer, mit Ausnahme von Vinylpyrrolidon/Dimethylaminoethylmethacrylat-Copolymer, aufweisen, verleihen dem behandelten Haaren einen festen Halt und gleichzeitig haarpflegende Eigenschaften, insbesondere eine gute Nasskämmbarkeit und eine effiziente Reduktion der statischen Aufladung des trockenen Haares, ohne das Haar zu belasten.

EP 1 502 577 A2

**Beschreibung**

Die Erfindung betrifft:

- ein haarfestigendes Mittel, wie es im Anspruch 1 umschrieben ist; und
- ein Verfahren zu dessen Herstellung, wie es im Anspruch 24 umschrieben ist.

Bevorzugte Ausführungformen sind in den abhängigen Ansprüchen umschrieben

Haarstylingprodukte dienen dazu, die Haare in Form zu bringen sowie das Haar zu pflegen. Isobutylen/Ethylmaleimid/Hydroxyethylmaleimid-Copolymere sind in der Lage, der Frisur bereits in niedriger Konzentration einen festen Halt zu verleihen, der auch bei hoher Luftfeuchtigkeit beständig ist. Sie sind in der Lage, der Frisur bereits in niedriger Konzentration einen festen Halt zu verleihen, welcher auch bei hoher Luftfeuchtigkeit beständig ist. Sie haben jedoch den Nachteil, dass sie dem nassen Haar keine gute Kämmbarkeit verleihen, die statische Aufladung des trockenen Haares nicht vermindern und im trockenen Haar einen steifen, unnatürlichen Haargriff hinterlassen. Kationische Polymere oder Polymere mit kationischen Gruppen sind sehr effiziente Kämmbarkeitsverbesserer, verhindern effizient die statische Aufladung der Haare und verleihen dem Haar einen natürlichen Griff, jedoch liegt ihre Festigungskraft wesentlich unter derjenigen der oben erwähnten Terpolymere. Deshalb muss zur Erzielung derselben Festigung ein erheblich höherer Gehalt an kationischen Polymeren eingesetzt werden, was zu einer übermässigen Haarbelastung führt. Zudem neigen kationische Polymere dazu, sich über mehrere Haarwäschen hinweg am Haar anzureichern, was dazu führen kann, dass das Haar einen stumpfen und belasteten Aspekt erhält.

Um in polymerhaltigen Stylingprodukten mit oben erwähnten Terpolymeren die Nasskämmbarkeit von Haaren zu verbessern und einen natürlicheren Haargriff zu erzielen, werden im allgemeinen Zusatzstoffe, wie Öle oder kationische niedrigmolekulare Stoffe, verwendet.

Werden die oben erwähnten Terpolymere - zum Zweck der Verbesserung der pflegenden Eigenschaften - mit kationischen Polymeren oder mit kationischen Zusatzstoffen versetzt, erfolgt jedoch bei anwendungsgerechten pH-Werten eine nicht erwünschte spontane Ausfällung des Polymers. Die Öle, insbesondere auch Siliconöle, besitzen den Nachteil, dass sie als Weichmacher die Festigung der Frisur herabsetzen und zusätzlich dem Haar eine störende Belastung abgeben.

Aufgabe der vorliegenden Erfindung war es somit, ein haarfestigendes Haarbehandlungsmittel zu schaffen, welches den behandelten Haaren einen festen Halt verleiht und gleichzeitig haarpflegende Eigenschaften, insbesondere eine gute Nasskämmbarkeit und eine effiziente Reduktion der statischen Aufladung des trockenen Haares, besitzt, ohne das Haar zu belasten, und welches die oben genannten Nachteile nicht aufweist.

Diese Aufgabe wurde durch die in den Ansprüchen 1 und 24 definierten Massnahmen gelöst.

Aus der Literatur bekannt ist diesbezüglich die Kombination eines Isobutylen/Ethylmaleimid/Hydroxyethylmaleimid-Copolymers (Aquaflex FX-64® der Firma International Speciality Products) mit einem Vinylpyrrolidon/Dimethylaminoethylmethacrylat-Copolymer (Copolymer 845® der International Specialty Products) aus einer Anwendungspräsentation der International Specialty Products. Der Nachteil dieser Kombination liegt darin, dass Copolymere aus Vinylpyrrolidon und Dimethylaminoethylmethacrylat zwar eine gute Kämmbarkeit und einen guten Haargriff hervorrufen, jedoch in Kombination mit dem Isobutylen/Ethylmaleimid/Hydroxyethylmaleimid-Terpolymer die Festigungswirkung des Terpolymers abschwächen und die Zuverlässigkeit der Festigung bei hohen Luftfeuchtigkeiten vermindern.

Gegenstand der Erfindung ist somit ein haarfestigendes Mittel mit einem Gehalt an:

(A) mindestens einem Isobutylene/Ethylmaleimid/Hydroxyethylmaleimid-Terpolymer, und
(B) mindestens einem mit (A) verträglichen kationischen Polymer, mit Ausnahme von Vinylpyyrolidon/Dimethylaminoethylmethacrylat-Copolymer.

Die Polymere (A) und (B) werden so gewählt, dass sie miteinander verträglich sind. Unter dem Begriff "verträglich" wird verstanden, dass bei Mischung von Polymer (A) mit Polymer (B) keine spontane Ausklumpung erfolgt. Die Polymermischung soll im Haarbehandlungsmittel so vorliegen, dass das erfindungsgemässe haarfestigende Mittel in handelsüblichen Applikationsformen leicht auf dem Haar verteilbar ist und einen unsichtbaren, festigenden Film auf dem Haar ausbildet, ohne dass auf dem Haar eine Verklumpung festzustellen ist.

Überraschenderweise wurde gefunden, dass die erfindungsgemässe Kombination von (A) und (B) zu einem Haarbehandlungsmittel führt, das sowohl einen festen, auch bei hoher Luftfeuchtigkeit zuverlässigen Halt, als auch eine gute Kämmbarkeit im nassen Haar ergibt, sowie im trockenen Haar einen natürlichen Haargriff erzeugt.

Das Mittel kann Form eines Aerosol- oder Non-Aerosol-Haarsprays, eines Haarschaumes, eines Haargeles, eines Haarwachses, einer Haarcreme oder einer Haarlotion vorliegen.

Die Menge des Terpolymers (A) beträgt im erfindungsgemässen haarfestigenden Mittel vorzugsweise 0,01 bis 20 Gewichtsprozent, insbesondere 0,2 bis 5 Gewichtsprozent. Die Menge des kationischen Polymers (B) beträgt im erfindungsgemässen haarfestigenden Mittel vorzugsweise 0,01 bis 10 Gewichtsprozent, insbesondere 0,1 bis 3 Gewichtsprozent. Vorzugsweise ist im erfindungsgemässen haarfestigenden Mittel der Gehalt an (A) mindestens gleich hoch oder höher als der Gehalt an (B).

Das erfindungsgemässe haarfestigende Mittel wird vorzugsweise in einem wässrigen oder in einem wässrig-alkoholischen Milieu konfektioniert. Als Alkohole können insbesondere die für kosmetische Zwecke üblicherweise verwendeten niederen Alkohole mit 1 bis 4 Kohlenstoffatomen, wie zum Beispiel Ethanol und Isopropanol, eingesetzt werden. Des weiteren können Lösungsmittel oder ein Gemisch aus Lösungsmitteln mit einem Siedepunkt unter 400 °C in einer Menge von 0,1 bis 90 Gewichtsprozent, vorzugsweise von 1 bis 50 Gewichtsprozent, eingesetzt werden. Besonders geeignet sind unverzweigte oder verzweigte Kohlenwasserstoffe, wie Pentan, Hexan, Isopentan, und cyclische Kohlenwasserstoffe, wie Cyclopentan und Cyclohexan. Weitere besonders bevorzugte wasserlösliche Lösungsmittel sind Glycerin und Propylenglykol in einer Menge von bis zu 30 Gewichtsprozent.

Das erfindungsgemässe haarfestigende Mittel kann in einem pH-Bereich von mehr als 6,0 vorliegen. Besonders bevorzugt ist der pH-Bereich zwischen 6,0 und 8. Liegt das erfindungsgemässe haarfestigende Mittel im sauren Bereich vor, so kann es organische oder anorganische Säuren enthalten, wie beispielsweise Ameisensäure, Weinsäure, Äpfelsäure, Maleinsäure, Fumarsäure, Ameisensäure, Pyrrolidoncarbonsäure, Citronensäure, Schwefelsäure, Essigsäure, Salzsäure oder Phosphorsäure.

Das Terpolymer (A) wird beispielsweise von der Firma International Specialty Products unter der Handelsbezeichnung "Aquaflex FX64®" vertrieben (INCI-Bezeichnung: Isobutylene/Ethylmaleimide/Hydroxyethylmaleimide Copolymer) in den Handel gebracht.

Das kationische Polymer (B) ist ein natürliches oder synthetisches Homo- oder Copolymer, wobei das Polymer vorzugsweise mindestens eine quaternäre oder eine quaternisierbaren Aminogruppe enthält. Die quaternäre oder quaternisierbare Aminogruppe des kationischen Polymers liegt entweder in der Polymerkette oder vorzugsweise als Substituent an einem oder mehreren Monomeren vor.

Geeignete Monomere eines synthetischen kationischen Polymers (B), welches quaternäre oder quaternisierbare Aminogruppen aufweist, sind ungesättigte, radikalisch polymerisierbare Verbindungen, welche mindestens eine quaternäre oder eine quaternisierbare Aminogruppe tragen, insbesondere ammoniumsubstituierte Vinylmonomere oder quaternisierte Derivate von Carboxyvinylmonomeren, wie z.B. quaternisierte Acrylamide oder Methacrylamide. Beispiele hierfür sind Acrylamidoalkyltrialkylammoniumhalogenide oder Methacrylamidoalkyltrialkylammoniumhalogenide, Trialkylmethacryloxyalkylammoniumhalogenide, Trialkylacryloxyalkylammoniumhalogenide, Dialkyldiallylammoniumhalogenide oder quaternäre Vinylammoniummonomere mit cyclischen, kationische Stickstoffe enthaltenden Gruppen, wie Pyridinium, Imidazolium oder quaternäre Pyrrolidone, z.B. Alkylvinylimidazolium-, Alkylvinylpyridinium-, oder Alyklvinylpyrrolidon-Salze.

Die Alkylgruppen dieser Monomere sind vorzugsweise niedere Alkylgruppen, wie zum Beispiel C1- bis C7-Alkylgruppen, insbesondere C1- bis C3-Alkylgruppen. Bevorzugt sind Acrylamidopropyltrimethylammoniumchlorid und Methacrylamidopropyltrimethylammoniumchlorid.

Beispiele für synthetische kationische Polymere, welche als Komponente (B) geeignet und besonders bevorzugt sind, sind Copolymere aus Vinylcaprolactam, Vinylpyrrolidon und Methylvinylimidazoliummethosulfat (INCI-Bezeichnung: Polyquaternium-46), wie sie beispielswiese von der Firma BASF unter der Handelsbezeichnung "Luviquat Hold®" vertrieben werden, Copolymere aus Vinylpyrrolidon und Dimethylaminopropylacrylamid oder -methycrylamid (INCI-Bezeichnung: Vinylpyrrolidon/DMAPA Acrylates Copolymer), wie sie beispielsweise von der Firma International Specialty Products unter der Handelsbezeichnung "Styleze CC-10®" in den Handel gebracht werden, Copolymere aus Vinylpyrrolidon, Dimethylaminopropylmethacrylamid und Methacryloylaminopropyllauryldimoniumchlorid (INCI-Bezeichnung: Polyquaternium-55), wie sie beispielsweise von der Firma International Specialty Products unter der Handelsbezeichnungen "Styleze W-10®" und "Styleze W-20®" vertrieben werden.

Beispiele natürliche kationische Polymere, welche als Komponente (B) geeignet und besonders bevorzugt sind, sind Copolymere aus Hydroxyethylcellulose und Diallyldimethylammoniumchlorid (INCI-Bezeichnung: Polyquaternium-4), wie sie beispielsweise von der Firma National Starch unter der Handelsbezeichnungen "Celquat H-100" und "Celquat L-200" in den Handel gebracht werden.

Eine bevorzugte Ausführungsform des erfindungsgemässen haarfestigenden Mittels enthält zusätzlich kationische Kämmbarkeitsverbesserer, insbesondere kationische Tenside oder kationische Siliconverbindungen. Die Kämmbarkeitsverbesserer werden zweckmässig in einer Menge von 0,05 bis 10 Gewichtsprozent, vorzugsweise von 0,1 bis 3 Gewichtsprozent, eingesetzt. Die kationischen Kämmbarkeitsverbesserer zeichnen sich dadurch aus, dass sie in Kombination mit dem Terpolymer (A) alleine im bevorzugten pH-Bereich zu Ausfällungen führen, in Zusammensetzungen gemäss dem erfindungsgemässen haarfestigenden Mittel jedoch keine Ausfällungen hervorrufen.

Geeignete kationische Tenside besitzen die allgemeine Formel $[NR^1R^2R^3R^4]^+$ $X^-$, wobei die Reste $R^1$, $R^2$, $R^3$ und $R^4$ unabhängig voneinander eine aliphatische Gruppe mit 1 bis 22 Kohlenstoffatomen oder eine aromatische, Alkoxy-, Polyoxyalkylen-, Alkylamido-, Hydroxyalkyl-, Aryl- oder Alkylarylgruppe mit bis zu 22 Kohlenstoffatomen bedeuten. X ist ein salzbildendes Anion, beispielsweise Halogen, wie Chlorid oder Bromid, Acetat, Citrat, Lactat, Glykolat, Phosphat, Nitrat, Sulfat oder Alkylsulfat. Die langkettigen aliphatischen Gruppen, d.h. solche mit rund 12 oder mehr Kohlenstoffatomen, können gesättigt oder ungesättigt sein. Bevorzugt sind kationische Tenside mit entweder einer langkettigen und drei kurzkettigen oder mit zwei langkettigen und zwei kurzkettigen aliphatischen Gruppen, wobei die langkettigen Gruppen 12 bis 22, vorzugsweise 16 bis 22 Kohlenstoffatome, und die kurzkettigen Gruppen 1 bis 3, vorzugsweise 1 bis 2 Kohlenstoffatome, aufweisen. Bevorzugte kationische Tenside sind Cetyltrimethylammoniumchlorid, Behenyltrimethylammoniumchlorid, Stearyltrimethylammoniumchlorid, Stearyldimethylbenzylammoniumchlorid, Cetylpyridiniumchlorid, Ditallowdimethylammoniumchlorid, Monotallowtrimethylammoniumchlorid und Dicetyldimethylammoniumchlorid sowie deren Gemische.

Geeignete kationische Siliconverbindungen sind insbesondere mit quaternären Aminogruppen endfunktionalisierte Siliconpolymere, beispielsweise die unter den Handelsbezeichnungen "Abil® Quat 3270", "Abil® Quat 3272" und "Abil Quat 3474®" von der Firma Goldschmidt in den Handel gebrachten Produkte (INCI-Bezeichnung Quaternium-80).

Bevorzugte Ausführungsformen des erfindungsgemässen haarfestigenden Mittels enthalten als weitere Komponente 0,01 bis 10 Gewichtsprozent, vorzugsweise 0,5 bis 5 Gewichtsprozent, mindestens eines synthetischen oder natürlichen filmbildenden nichtionischen haarfestigenden Polymers. Die haarfestigenden Polymere können einzeln oder in einem Gemisch eingesetzt werden. Unter filmbildenden, haarfestigenden Polymeren sollen solche Polymere verstanden werden, welche allein in 0,1- bis 5%iger wässriger, alkoholischer oder wässrig-alkoholischer Lösung angewandt, in der Lage sind, auf dem Haar einen Polymerfilm abzuscheiden und auf diese Weise das Haar zu festigen.

Geeignete synthetische, nichtionische, filmbildende, haarfestigende Polymere sind zum Beispiel Homopolymere des Vinylpyrrolidons, wie sie beispielsweise unter den Handelsbezeichnungen "Luviskol K®" von der Firma BASF (Deutschland) oder "PVP-K®" von der Firma international Specialty Products (USA) in den Handel gebracht werden.

Weitere geeignete synthetische filmbildende, nichtionische haarfestigende Polymere sind zum Beispiel:

- Copolymerisate aus Vinylpyrrolidon und Vinylacetat, wie sie beispielsweise unter den Handelsbezeichnungen "Luviskol VA®" von der Firma BASF (Deutschland) in den Handel gebracht werden;
- Vinylpyrrolidon/Vinylacetat/Vinylpropionat-Terpolymere, wie sie beispielsweise unter der Handelsbezeichnung "Luviskol VAP®" von der Firma BASF (Deutschland) in den Handel gebracht werden;
- Polyacrylamide, wie sie beispielsweise unter den Handelsbezeichnungen "Akypomine P 191®" von der Firma CHEM-Y (Deutschland) oder "Sepigel 305®" von der Firma SEPPIC (USA) in den Handel gebracht werden;
- Polyvinylalkohole, wie sie beispielsweise unter den Handelsbezeichnungen "Elvanol®" von der Firma Du Pont oder "Vinol 523/540®" von der Firma Air Porducts (USA) in den Handel gebracht werden; ferner
- hochmolekulares Polyethylenglykol oder hochmolekulare Copolymere von Ethylenglykol mit Propylenglykol mit festigenden Eigenschaften, wie sie beispielsweise unter den Handelsbezeichnungen "Lipoxol 1000®" von der Firma HÜLS AG (Deutschland), "Pluracol E 4000®" von der Firma BASF (Deutschland) oder "Upiwax 20.000®" von der Firma Universal Preserv-A-Chem Inc, (Edison, NJ, USA) in den Handel gebracht werden.

Geeignete natürliche filmbildende Polymere mit haarfestigender Wirkung sind zum Beispiel modifizierte Korn-Stärken, wie sie beispielsweise von der Firma National Starch unter der Handelsbezeichnung "Amaze®" (INCI-Bezeichnung: Corn Starch Modified) in den Handel gebracht werden, oder verschiedene Saccharidtypen, wie zum Beispiel Polysaccharide oder Gemische aus Oligo-, Mono- und Disacchariden, welche beispielsweise unter dem Handelsnamen "C-Pur®" von der Firma Cerestar (Brüssel, Belgien), in den Handel gebracht werden.

Weitere geeignete natürliche Polymere sind chinesisches Balsamharz und Cellulosederivate, zum Beispiel Hydroxypropylcellulose mit einem Molekulargewicht von 30'000 bis 50'000 g/mol, welche beispielsweise unter der Handelsbezeichnung "Nisso SL®" von der Firma Lehmann & Voss (Deutschland) in den Handel gebracht wird.

Des Weiteren können Polymere mit verdickender Wirkung eingesetzt werden. Verdickende Polymere sind Polymere, welche bei einem Feststoffgehalt von 1 % in Wasser nach bestimmungsgemässer Einarbeitung (z.B. in Neutralisationsschritten) eine Viskosität von mindestens 1000 mPa·s aufweisen. Besonders bevorzugte verdickende Polymere sind beispielsweise Acrylat/Steareth-20-methacrylat-Copolymere, welche unter der Handelsbezeichnung "Aculyn 22®" von der Firma Rohm und Haas in den Handel gebracht werden, sowie Acrylat/Beheneth-25-methacrylat-Copolymere, welche unter der Handelsbezeichnung "Aculyn 28®" von der Firma Rohm und Haas vertrieben werden.

Als weitere verdickende Polymere, die im erfindungsgemässen haarfestigenden Mittel enthalten sein können, sind Homopolymere der Acrylsäure mit einem Molekulargewicht von 2'000'000 bis 6'000'000 zu nennen, wie sie beispielsweise unter der Handelsbezeichnung "Carbopol®" von der Firma Noveon in den Handel gebracht werden. Weitere geeignete Verdicker sind beispielsweise das unter dem Handelsnamen "Carbopol ETD 2001®" von der Firma Noveon in den Handel gebrachte Produkt. Besonders bevorzugt werden die Copolymere der Acrylsäure oder der Methacrylsäure, wie sie zum Beispiel unter dem Handelsnamen "Carbopol 1342®" oder "Pemulen TR1®" von der Firma Goodrich (USA) in den Handel gebracht werden.

Als Neutralisationsmittel für die verdickenden Polymere oder als pH-Regulatoren für das Herstellverfahren können für kosmetische Zwecke geeignete organische oder anorganische Basen verwendet werden. Beispiele solcher Basen sind Aminoalkohole, wie z.B. Aminomethylpropanol (AMP), Triethanolamin oder Monoethanolamin, Ammoniak und NaOH.

Selbstverständlich kann das erfindungsgemässe haarfestigende Mittel auch weitere übliche kosmetische Zusätze enthalten, wie beispielsweise nichtfestigende nichtionische Polymere, z.B. Polyethylenglykole oder Copolymere aus Ethylenglykol und Propylenglykol; nichtfestigende anionische Polymere und nichtfestigende natürliche Polymere sowie deren Kombination in einer Menge von vorzugsweise 0,01 bis 50 Gewichtsprozent; Parfümöle in einer Menge von vorzugsweise 0,01 bis 5 Gewichtsprozent; Trübungsmittel, wie zum Beispiel Ethylenglykoldistearat, in einer Menge von vorzugsweise 0,01 bis 5 Gewichtsprozent; Netzmittel oder Emulgatoren aus den Klassen der anionischen, amphoteren oder nichtionogenen oberflächenaktiven Substanzen, wie Fettalkoholsulfate, ethoxylierte Fettalkohole, Fettsäurealkanolamide, wie zum Beispiel die Ester der hydrierten Rizinusölfettsäuren, in einer Menge von vorzugsweise 0,1 bis 30 Gewichtsprozent; ferner Feuchthaltemittel, Farbstoffe, Lichtschutzmittel, Antioxidantien, Glanzgeber und Konservierungsstoffe in einer Menge von vorzugsweise 0,01 bis 10 Gewichtsprozent.

Als weitere Zusätze sind geeignete Siliconpolymere einsetzbar: Polydimethylsiloxan (INCI-Bezeichnung: Dimethicon), α-Hydro-ω-hydroxypolyoxydimethylsilylen (INCI-Bezeichnung: Dimethiconol), cyclisches Dimethylpolysiloxan (INCI-Bezeichnung: Cyclomethicon), Trimethyl(octadecyloxy)silan (INCI-Bezeichnung: Stearoxytrimethylsilan), Dimethylsiloxan/Glykol-Copolymer (INCI-Bezeichnung: Dimethicon Copolyol), Dimethylsiloxan/Aminoalkylsiloxan-Copolymer mit Hydroxyendgruppen (INCI-Bezeichnung: Amodimethicon), Monomethylpolysiloxan mit Laurylseitenketten und Polyoxyethylenund/oder Polyoxypropylenendketten, (INCI-Bezeichnung: Laurylmethicon Copolyol), Dimethylsiloxan/Glykol-Copolymeracetat (INCI-Bezeichnung: Dimethiconcopolyol Acetat) Dimetylsiloxan/Aminoalkylsiloxan-Copolymer mit Trimethylsilylendgruppen (INCI-Bezeichnung: Trimethylsilylamodimethicon). Bevorzugte Siliconpolymere sind: Dimethicone, welche beispielsweise von der Firma Wacker (München/Deutschland), unter der Handelsbezeichnung "Siloxane F-221®" oder von der Firma Dow Corning Europe (Brüssel/ Belgien), unter der Handelsbezeichnung "Dow Corning Fluid 200/ 0,65 cs®" in den Handel gebracht werden; Cyclomethicone, wie sie beispielsweise unter den Handelsbezeichnungen "Dow Corning 244 Fluid®" von der Firma Dow Corning Europe (Brüssel/Belgien), oder "Abil K4®" von der Firma Goldschmidt (Deutschland) in den Handel gebracht werden; Dimethiconole, wie sie beispielsweise unter den Handelsbezeichnungen "Silicone Fluid F-212®" von der Firma Wacker (Deutschland) oder "Unisil® SF-R®" von der Firma Universal Preserv-A-Chem Inc, (Edison, NJ, USA) in den Handel gebracht werden.

Die vorstehend in Klammern angegebenen Bezeichnungen entsprechen der INCI-Nomenklatur (International Cosmetic Ingredients), wie sie zur Kennzeichnung kosmetischer Wirk- und Hilfsstoffe bestimmt sind.

Auch Mischungen von Siliconpolymeren sind geeignet, wie zum Beispiel eine Mischung aus Dimethicon und Dimethiconol, wie sie beispielsweise unter der Handelsbezeichnung "Dow Corning 1501 Fluid®" von der Firma Dow Corning Europe (Belgien) vertrieben wird.

Das erfindungsgemässe haarfestigende Mittel kann in verschiedenen Applikationsformen Verwendung finden, wie beispielsweise in Aerosolzubereitungen als Schaum oder als Spray, des weiteren als Non-Aerosol, welches mittels einer Pumpe oder als "Pump and Spray" zum Einsatz kommt. Der Einsatz in üblichen Öl-in-Wasser- und Wasser-in-Öl-Emulsionen ist ebenso möglich wie in Anwendungsformen als Gel, Wachs, Lotion oder Mikroemulsion. Das erfindungsgemässe haarfestigende Mittel kann auch als färbendes oder pflegendes Haarbehandlungsmittel, wie zum Beispiel als Farbfestiger und Haarspülung, formuliert sein.

Wenn das erfindungsgemässe haarfestigende Mittel in Form eines Aerosol-Haarsprays oder Aerosol-Haarlackes vorliegt, so enthält es zusätzlich zweckmässig 15 bis 85 Gewichtsprozent, vorzugsweise 25 bis 75 Gewichtsprozent, eines Treibmittels und wird in einem Druckbehälter abgefüllt. Als Treibmittel sind beispielsweise niedere Alkane, wie zum Beispiel n-Butan, i-Butan und Propan, oder auch deren Gemische mit Dimethylether, sowie ferner bei den in Betracht kommenden Drücken gasförmig vorliegende Treibmittel, wie beispielsweise $N_2$, $N_2O$ und $CO_2$ sowie Gemische der vorstehend genannten Treibmittel geeignet.

Wenn das erfindungsgemässe Mittel in Form eines versprühbaren Non-Aerosol-Haarsprays oder eines Non-Aerosol-Haarlacks vorliegt, so wird es mit Hilfe einer geeigneten mechanisch betriebenen Sprühvorrichtung versprüht. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemässe kosmetische Mittel unter Druck abgefüllt wird, verwendet werden, wobei sich der elastische Behälter ausdehnt und aus dem das Mittel infolge der Kontraktion des elastischen Behälters bei Öffnen des Sprühventils kontinuierlich abgegeben wird,.

Wenn das erfindungsgemässe haarfestigende Mittel in Form eines festigenden Haarschaumes (Mousse) vorliegt, so ist mindestens eines der Polymere schaumbildend, oder es enthält zusätzlich mindestens eine für diesen Zweck bekannte, übliche schaumgebende Substanz. Das Mittel wird mit oder ohne Hilfe von Treibgasen oder chemischen Treibmitteln verschäumt, als Schaum in das Haar eingearbeitet und ohne Ausspülen im Haar belassen. In diesem Falle ist eine mechanische Vorrichtung zum Verschäumen der Zusammensetzung vorgesehen. Unter mechanischen Schäumvorrichtungen sind solche Vorrichtungen zu verstehen, welche das Verschäumen einer Flüssigkeit mit oder ohne Verwendung eines Treibmittels ermöglichen. Als geeignete mechanische Schäumvorrichtung kann beispielsweise ein handelsüblicher Pumpschäumer oder ein Aerosolschaumkopf verwendet werden.

Wenn das erfindungsgemässe haarfestigende Mittel in Form eines festigenden Haargels vorliegt, so enthält es zusätzlich mindestens eine gelbildende Substanz, beispielsweise die oben aufgeführten Verdickungsmittel in einer Menge von vorzugsweise 0,05 bis 10 Gewichtsprozent, insbesondere von 0,1 bis 2 Gewichtsprozent. Die Viskosität des Gels beträgt bei 25°C vorzugsweise 500 bis 50'000 cSt, insbesondere 1'000 bis 15'000 cSt.

Wenn das erfindungsgemässe haarfestigende Mittel in Form eines Haarwachses vorliegt, so enthält es zusätzlich wasserunlösliche Fettstoffe oder fett- bzw. wachsähnliche Stoffe in einer Menge von vorzugsweise 0,5 bis 30 Gewichtsprozent. Geeignete wasserunlösliche Stoffe sind beispielsweise Emulgatoren mit einem HLB-Wert unterhalb von 7, Siliconöle, Siliconwachse, Wachse, z.B. Wachsalkohole, Wachssäuren, Wachsester, sowie insbesondere natürliche Wachse, wie Bienenwachs, Carnaubawachs; Fettalkohole, Fettsäuren oder Fettsäureester.

Wenn das erfindungsgemässe haarfestigende Mittel in Form einer festigenden Haarlotion vorliegt, so liegt es als nicht-viskose Lösung, Dispersion oder Emulsion mit einem Gehalt von mindestens 10 Gewichtsprozent, vorzugsweise 20 bis 95 Gewichtsprozent, eines kosmetisch verträglichen Alkohols vor. Als Alkohole können insbesondere die für kosmetische Zwecke üblicherweise verwendeten niederen Alkohole mit 1 bis 4 Kohlenstoffatomen, wie zum Beispiel Ethanol und Isopropanol, eingesetzt werden.

Wenn das erfindungsgemässe haarfestigende Mittel in Form einer festigenden Haarcreme, beispielsweise einer Färbecreme, vorliegt, so liegt es vorzugsweise als Emulsion vor und enthält entweder zusätzlich viskositätsgebende Inhaltsstoffe, wie die oben genannten Verdicker, in einer Menge von 0,1 bis 10 Gewichtsprozent, oder es wird die erforderliche Viskosität und Konsistenz wird durch Micellbildung mit Hilfe von geeigneten Emulgatoren, Fettsäuren, Fettalkoholen oder Wachsen, in üblicher Weise aufgebaut.

Des Weiteren ist mit der erfindungsgemässen Polymerkombination die Herstellung von Konzentraten möglich, welche einen verringerten Wasser- oder Lösungsmittelgehalt aufweisen. Die Konzentrate werden nach Transport und gegebenenfalls Lagerung durch Zugabe der erforderlichen Menge Wasser oder Lösungsmittel in ein anwendungsfähiges Haarbehandlungsmittel überführt.

Unter Haarbehandlung soll die Behandlung des menschlichen Kopfhaares vor allem zum Zweck der Herstellung einer Frisur oder zur Pflege der Haare verstanden werden.

Das erfindungsgemässe haarfestigende Mittel wird angewendet, indem eine für die gewünschte Festigung ausreichende Menge in oder auf dem trockenen Haar oder nach der Haarwäsche in oder auf dem feuchten, handtuchgetrockneten Haar verteilt wird. Die anzuwendende Menge hängt von der Haarfülle ab und beträgt typischerweise 5 bis 30 g. Das Mittel wird vorzugsweise nicht ausgespült, verbleibt also auf dem Haar. Anschliessend wird das Haar gegebenenfalls durchgekämmt oder zur Frisur geformt und gegebenenfalls getrocknet. Die Frisurenerstellung kann aber auch bereits vor der Aufbringung des Mittels erfolgen, insbesondere wenn das Mittel in Form eines Sprays vorliegt.

Das erfindungsgemässe Verfahren zur Herstellung des erfindungsgemässen haarfestigenden Mittels umfasst die folgenden Schritte in der angegebenen Reihenfolge:

(a) das kationische Copolymer (B) wird in Wasser oder einem Wasser/Ethanol-Gemisch gelöst;
(b) das Gemisch wird durch Zugabe einer Base auf ein pH von mehr als 7,0, vorzugsweise von mehr als 8,0, eingestellt;
(c) das Terpolymer (A) wird portionenweise zugegeben, wobei sich zunächst eine Trübung bildet, die mit steigendem Gehalt an (A) teilweise oder ganz verschwindet; und
(d) gegebenenfalls werden die weiteren Zusatzstoffe zugegeben.

Die nachfolgenden Beispiele sollen den Gegenstand der Erfindung näher erläutern.

**Beispiele**

Soweit nichts anderes angegeben ist erfolgen nachstehend die Angaben entsprechend der INCI-Nomenklatur (International Cosmetic Ingredients), wie sie zur Kennzeichnung kosmetischer Wirk- und Hilfsstoffe bestimmt sind.

In den Beispielen werden die folgenden Rohstoffe eingesetzt:

| Rohstoff | INCI-Bezeichnung |
|---|---|
| Aquaflex FX-64 | Isobutylene/Ethylmaleimide/ Hydroxyethylmaleimide Copolymer |
| Celquat L-200 | Polyquaternium-4 |
| Luviquat Hold | Polyquaternium-46 |

(fortgesetzt)

| Rohstoff | INCI-Bezeichnung |
|---|---|
| Styleze CC-10 | Vinylpyrrolidone/DMAPA Acrylates Copolymer |
| Styleze 20 | Polyquaternium-55 |
| Aculyn 22 | Acrylates/Steareth-20 Methycrylate Copolymer |
| Aculyn 28 | Acrylates/Behenetz-25 Methacrylate Copolymer |
| Amaze | Corn Starch Modified |
| Amaze XT | Dehydroxanthan Gum |
| Carbopol Ultrez 21 | Acrylates/C10-30 Alkyl Acrylate Copolymer |

**Beispiel 1: Aerosolspray**

| | |
|---|---|
| Luviquat Hold | 3,00 g |
| Ethanol | 50,00 g |
| Wasser | 21,00 g |
| Aquaflex FX-64 | 6,00 g |
| Aminomethylpropanol | auf pH 6.5 bis 7 |
| Dimethylether | 20,00 g |
| Total | 100,00 g |

**Beispiel 2: Non-Aerosolspray und Haarlotion**

| | |
|---|---|
| Aquaflex FX-64 | 5,00 g |
| Celquat L-200 | 0,30 g |
| Ethanol | 60,00 g |
| Wasser | 34,70 g |
| NaOH | auf pH 6.5 bis 7 |
| Total | 100,00 g |

**Beispiel 3: Non-Aerosolspray mit kationischem Zusatzstoff**

| | |
|---|---|
| Aquaflex FX-64 | 5,00 g |
| Luviquat Hold | 0,30 g |
| Wasser | 94,60 g |
| Cetrimonium Chloride | 0,10 g |
| NaOH | auf pH 6.5-7 |
| Total | 100,00 g |

**Beispiel 4: Schaumfestiger**

| | |
|---|---|
| Aquaflex FX-64 | 7,00 g |
| Luviquat Hold | 3,00 g |

(fortgesetzt)

| Wasser | 81,80 g |
|---|---|
| Decyl Glucoside | 0,20 g |
| NaOH | auf pH 6,5 bis 7 |
| Propan/Butan 5,0 bar | 8,00 g |
| Total | 100,00 g |

**Beispiel 5: Schaumfestiger mit kationischem Zusatzstoff**

| Aquaflex FX-64 | 7,00 g |
|---|---|
| Luviquat Hold | 3,00 g |
| Wasser | 81,7 g |
| Decyl Glucoside | 0,20 g |
| NaOH | auf pH 6,5 bis 7 |
| Cetrimonium Chloride | 0,10 g |
| Propan/Butan 5,0 bar | 8,00 g |
| Total | 100,00 g |

**Beispiel 6: Schaumfestiger mit schaumgebendem Polymer**

| Aquaflex FX-64 | 7,00 g |
|---|---|
| Luviquat Hold | 3,00 g |
| Wasser | 81,90 g |
| NaOH | 0,10 g |
| Aculyn 22 | auf pH 6,5 bis 7 |
| Propan/Butan 5,0 bar | 8,00 g |
| Total | 100,00 g |

**Beispiel 7: Schaumfestiger**

| Aquaflex FX-64 | 7,00 g |
|---|---|
| Celquat L-200 | 0,50 g |
| Wasser | 84,30 g |
| Decyl Glucoside | 0,20 g |
| NaOH | auf pH 6,5 bis 7 |
| Propan/Butan 5,0 bar | 8,00 g |
| Total | 100,00 g |

**Beispiel 8: Schaumfestiger mit nichtionischem Polymer**

| Aquaflex FX-64 | 7,00 g |
|---|---|
| Celquat L-200 | 0,50 g |
| Amaze | 1,00 g |
| Wasser | 83,30 g |

(fortgesetzt)

| Decyl Glucoside | 0,20 g |
|---|---|
| NaOH | auf pH 6,5-7 |
| Propan/Butan 5,0 bar | 8,00 g |
| Total | 100,00 g |

**Beispiel 9: Schaumfestiger**

| Aquaflex FX-64 | 7,00 g |
|---|---|
| Styleze W-20 | 1,50 g |
| Wasser | 83,30 g |
| Decyl Glucoside | 0,20 g |
| NaOH | auf pH 6,5 bis 7 |
| Propan/Butan 5,0 bar | 8,00 g |
| Total | 100,00 g |

**Beispiel 10: Festiger-Gel**

| Aquaflex FX-64 | 5,00 g |
|---|---|
| Luviquat Hold | 2,00 g |
| Wasser | 92,20 g |
| Hydroxyethylcellulose | 0,80 g |
| NaOH | auf pH 6,5-7 |
| Total | 100,00 g |

**Beispiel 11: Festiger-Gel**

| Aquaflex FX-64 | 5,00 g |
|---|---|
| Luviquat Hold | 2,00 g |
| Wasser | 92,20 g |
| Carbopol Ultrez 10 | 0,80 g |
| Aminomethylpropanol | auf pH 6,5-7 |
| Total | 100,00 g |

**Vergleichsversuche von Haarbehandlungsmitteln mit konstantem Polymergehalt (3,0 Gewichtsprozent)**

In der folgenden Testreihe wurden haarfestigende Mittel mit einem Gehalt an einem der nachfolgend genannten Polymere sowie haarfestigende Mittel mit einem Gehalt an einer Zweierkombination der genannten Polymere untersucht, wobei die Polymere ausgewählt waren aus:

- einem Terpolymer gemäss (A) [Abkürzung: T]
- einem kationischen Polymer gemäss (B) [Abkürzung: K]
- einem nichtionischen Polymer [Abkürzung: Ni]

Es ergab sich folgende Kombinations-Matrix mit den Bezeichnungen A bis F für die nachfolgenden Rezepturen:

|  | T | K | Ni |
|---|---|---|---|
| **T** | A | D | E |
| **K** | D | B | F |
| **Ni** | E | F | C |

Die Formulierungen wurden auf das Haar aufgetragen und von jeweils 5 Personen unabhängig voneinander bezüglich der untenstehenden Kriterien beurteilt. Dabei reichte die Beurteilungsskala von Note 1 "sehr gut" bis Note 5 "unbrauchbar", wobei die angegebenen Werte aus Mittelwertbildungen stammen. Die Auswertung der Resultate zeigt klar, dass Formulierung B (Zweierkombination aus Terpolymer und kationischem Polymer) die vorteilhafteste Rezeptur darstellt.

|  | A | B | C | D | E | F |
|---|---|---|---|---|---|---|
| Aquaflex FX-64 (40 % in EtOH/$H_2$O) | 7,5 | 0,0 | 0,0 | 5,0 | 5,0 | 0,0 |
| Luviquat Hold (20 % in Wasser) | 0,0 | 15,0 | 0,0 | 5,0 | 0,0 | 10,0 |
| PVPNA 735 W (50 % in Wasser | 0,0 | 0,0 | 6,0 | 0,0 | 2,0 | 2,0 |
| Wasser | ad 100 | ad 100 | ad 100 | ad 100 | ad 100 | ad 100 |

A: Terpolymer alleine
B Kationisches Polymer alleine
C: Nichtionisches Polymer alleine
D: Kombination Terpolymer plus kationisches Polymer
E: Kombination nichtionisches Polymer plus Terpolymer
F: Kombination nichtionisches Polymer plus kationisches Polymer

**Beurteilung der Rezepturen A bis F**

Skala von 1 "ausgezeichnet" bis 5 "sehr schlecht"

|  | A | B | C | D | E | F |
|---|---|---|---|---|---|---|
| Griff nass (1) | 4,3 | 3,1 | 3,0 | 1,8 | 2,5 | 2,1 |
| Kämmbarkeit | 4,5 | 1,4 | 3,7 | 1,7 | 4,2 | 1,7 |
| Klebrigkeit | 3,1 | 1,2 | 2,2 | 1,3 | 2,9 | 1,5 |
| Stärke des Haltes | 1,2 | 2,9 | 2,5 | 1,2 | 1,3 | 2,8 |
| Langzeithalt der Frisur | 1,5 | 3,2 | 4,1 | 1,4 | 3,8 | 2,5 |
| Elastizität/Volumen | 3,6 | 1,5 | 3,8 | 2,1 | 4,1 | 2,2 |
| Rauheit des Haares | 3,3 | 1,2 | 3,1 | 1,8 | 2,7 | 2,9 |
| Belastung/Rückstände | 2,0 | 1,0 | 2,2 | 1,8 | 3,1 | 2,5 |
| Schlechtester Wert | 4,3 | 3,2 | 4,1 | 2,1 | 4,2 | 2,8 |
| Durchschnittswert | 2,9 | 1,9 | 3,1 | 1,6 | 3,1 | 2,3 |

(1) positiv: leicht gleitend; negativ: schmierig oder stumpf

**Patentansprüche**

1. Haarfestigendes Mittel, **gekennzeichnet durch** einen Gehalt an:

   (A) mindestens einem Isobutylen/Ethylmaleimid/Hydroxyethylmaleimid-Terpolymer; und
   (B) mindestens einem mit (A) verträglichen kationischen Polymer, mit Ausnahme von Vinylpyrrolidon/Dimethylaminoethylmethacrylat-Copolymer.

2. Haarfestigendes Mittel nach Anspruch 1, **dadurch gekennzeichnet, dass** die Menge des Terpolymers (A) 0,01 bis 20 Gewichtsprozent beträgt.

3. Haarfestigendes Mittel nach Anspruch 2, **dadurch gekennzeichnet, dass** die Menge des Terpolymers (A) 0,2 bis 5 Gewichtsprozent beträgt.

4. Haarfestigendes Mittel nach einem der Ansprüche 1 bis 3, **dadurch gekennzeichnet, dass** die Menge des kationischen Polymers (B) 0,01 bis 10 Gewichtsprozent beträgt.

5. Haarfestigendes Mittel nach Anspruch 4, **dadurch gekennzeichnet, dass** die Menge des kationischen Polymers (B) 0,1 bis 3 Gewichtsprozent beträgt.

6. Haarfestigendes Mittel nach einem der Ansprüche 1 bis 5, **dadurch gekennzeichnet, dass** es in einem wässrigen oder wässrig-alkoholischem Milieu konfektioniert ist.

7. Haarfestigendes Mittel nach einem der Ansprüche 1 bis 6, **dadurch gekennzeichnet, dass** es ein pH von 6,0 bis 8 aufweist.

8. Haarfestigendes Mittel nach einem der Ansprüche 1 bis 7, **dadurch gekennzeichnet, dass** das kationische Polymer (B) mindestens eine quaternäre oder quaternärisierbare Amiogruppe enthält.

9. Haarfestigendes Mittel nach einem der Ansprüche 1 bis 8, **dadurch gekennzeichnet, dass** das kationische Polymer (B) das Copolymer von Hydroxyethylcellulose und Diallylammoniumchlorid ist.

10. Haarfestigendes Mittel nach einem der Ansprüche 1 bis 9, **dadurch gekennzeichnet, dass** es ausserdem einen kationischen Kämmbarkeitsverbesserer (C) enthält.

11. Haarfestigendes Mittel nach Anspruch 10, **dadurch gekennzeichnet, dass** der Kämmbarkeitsverbesserer (C) ein kationisches Tensid ist.

12. Haarfestigendes Mittel nach Anspruch 10 oder 11, **dadurch gekennzeichnet, dass** die Menge des Kämmbarkeitsverbesserers (C) 0,05 bis 10 Gewichtsprozent beträgt.

13. Haarfestigendes Mittel nach Anspruch 12, **dadurch gekennzeichnet, dass** die Menge des Kämmbarkeitsverbesserers (C) 0,1 bis 3 Gewichtsprozent beträgt.

14. Haarfestigendes Mittel nach Anspruch 11, **dadurch gekennzeichnet, dass** das kationische Tensid die allgemeine Formel $[NR^1R^2R^3R^4]^+$ $X^-$ aufweist, wobei die Reste $R^1$, $R^2$, $R^3$ und $R^4$ unabhängig voneinander eine aliphatische Gruppe mit 1 bis 22 Kohlenstoffatomen oder eine aromatische, Alkoxy-, Polyoxyalkylen-, Alkylamido-, Hydroxyalkyl-, Aryl- oder Alkylarylgruppe mit bis zu 22 Kohlenstoffatomen und X ein salzbildendes Anion bedeuten.

15. Haarfestigendes Mittel nach Anspruch 14, **dadurch gekennzeichnet, dass** das Tensid eine langkettige Gruppe mit 12 bis 22 Kohlenstoffatomen und drei kurzkettige Gruppen mit 1 bis 3 Kohlenstoffatomen aufweist.

16. Haarfestigendes Mittel nach Anspruch 14, **dadurch gekennzeichnet, dass** das Tensid zwei langkettige Gruppen mit 12 bis 22 Kohlenstoffatomen und zwei kurzkettige Gruppen mit 1 bis 3 Kohlenstoffatomen aufweist.

17. Haarfestigendes Mittel nach einem der Ansprüche 1 bis 16, **dadurch gekennzeichnet, dass** es ausserdem mindestens eines synthetischen oder natürlichen filmbildenden nichtionischen haarfestigenden Polymers (D) enthält.

18. Haarfestigendes Mittel nach Anspruch 17, **dadurch gekennzeichnet, dass** die Menge des haarfestigenden Polymers (D) 0,01 bis 10 Gewichtsprozent beträgt.

19. Haarfestigendes Mittel nach Anspruch 18, **dadurch gekennzeichnet, dass** die Menge des haarfestigenden Polymers (D) 0,5 bis 5 Gewichtsprozent beträgt.

20. Haarfestigendes Mittel nach einem der Ansprüche 1 bis 19, **dadurch gekennzeichnet, dass** es ausserdem ein Polymer mit verdickender Wirkung (E) enthält.

21. Haarfestigendes Mittel nach einem der Ansprüche 1 bis 20, **dadurch gekennzeichnet, dass** es als Aerosol-Haarspray oder Aerosol-Haarlack konfektioniert ist.

**22.** Haarfestigendes Mittel nach einem der Ansprüche 1 bis 20, **dadurch gekennzeichnet, dass** es als versprühbarer Non-Aerosol-Haarspray oder Non-Aerosol-Haarlack konfektioniert ist.

**23.** Haarfestigendes Mittel nach einem der Ansprüche 1 bis 20, **dadurch gekennzeichnet, dass** es als Haarschaum, Haargel, Haarwachs, Haarlotion oder Haarcreme mit festigenden Eigenschaften konfektioniert ist.

**24.** Verfahren zur Herstellung eine haarfestigenden Mittels nach einem der Ansprüche 1 bis 20, **dadurch gekennzeichnet, dass** es die folgenden Schritte in der angegebenen Reihenfolge umfasst:

(a) das kationische Copolymer (B) wird in Wasser oder einem Wasser/Ethanol-Gemisch gelöst;
(b) das Gemisch wird durch Zugabe einer Base auf ein pH von mehr als 7,0 eingestellt;
(c) das Terpolymer (A) wird portionenweise zugegeben: und
(d) gegebenenfalls werden die weiteren Zusatzstoffe (C), (D), (E) zugegeben.

**25.** Verfahren nach Anspruch 24, **dadurch gekennzeichnet, dass** im Verfahrensschritt (b) ein pH von mehr als 8,0 eingestellt wird.